# NEUE EUROPÄISCHE PATENTSCHRIFT

(11) **EP 0 324 947 B2**
(45) Veröffentlichungstag und Bekanntmachung des Hinweises auf die Entscheidung über den Einspruch: **20.08.1997**
(45) Hinweis auf die Patenterteilung: 04.11.1992
(21) Anmeldenummer: 88120859.9
(22) Anmeldetag: 14.12.1988
(51) Int. Cl.: A61K 31/195, A61K 45/06

(54) **Synergistische Kombination von Decarboxylasehemmern und L-Dopa-Pellets**
Synergistic combination of decarboxylase inhibitors and L-dopa pellets
Combinaison synergique d'inhibiteurs de décarboxylase et de pilules de L-Dopa

(30) Priorität: 31.12.1987 DE 3744646
(43) Veröffentlichungstag der Anmeldung: 26.07.1989
(73) Patentinhaber: ASTA Medica Aktiengesellschaft, D-01277 Dresden (DE)
(72) Erfinder: Albring, Manfred, Dr., D-1000 Berlin 27 (DE); Hettche, Helmut, Dr., D-6057 Dietzenbach (DE)

(56) Entgegenhaltungen:
- EP-A- 0 147 780
- EP-A- 0 269 236
- FR-A- 2 512 676
- US-A- 3 557 292
- US-A- 3 769 424
- Arzneiformenlehre (Dr. List, Dr. Müller, Dr. Nürnberg) 4. Auflage 1985, S.84-85
- H. Sucker et al. "Pharmazeutische Technologie", G. Thieme Verlag, Stuttgart, 1978, Seiten 155, 424-427
- W.A. Ritschel "Die Tablette", Der pharmazeutische Betrieb, Editio kantor KG, Aulendorf, 1966, Seite 213
- R. Voigt "Lehrbuch der pharmazeutischen Technologie", 6. Auflage, Seiten 198-199

## Beschreibung

Decarboxylasehemmer sind Arzneimittelwirkstoffe mit der Eigenschaft, periphere Decarboxylasen (das heißt im Darm und Blutkreislauf vorhandene Decarboxylasen) zu blockieren. Als Decarboxylasehemmer kommen in Frage Benserazid (DL-Serin-2-(2,3,4-trihydroxybenzyl)-hydrazid),Carbidopa ( (-)-L-α-Hydrazino-3,4-dihydroxy-α-methylhydrozimtsäure), L-Serin-2-(2,3,4-trihydroxybenzyl)-hydrazid, Glycin-2-(2,3,4-trihydroxybenzyl)-hydrazid, L-Tyrosin-2-(2,3,4-trihydroxybenzyl)hydrazid, insbesondere Benserazid.

L-Dopa ( (S)-2-Amino-3-(3,4-dihydroxyphenyl)propionsäure) ist ein Arzneimittelwirkstoff mit ausgeprägter Antiparkinson-Wirkung.

Die Kombination aus dem Decarboxylasehemmer Benserazid und L-Dopa ist seit 1960 bekannt. Hierbei liegt das L-Dopa nicht in Pelletform vor. Diese Formulierung hat den Nachteil, daß es bei der Therapie der Parkinsonschen Krankheit mit dieser Kombination zu starken Schwankungen des Plasmaspiegels und dadurch bedingten erheblichen Responsefluktuationen kommt, zum Beispiel zu den sogenannten on-off-Symptomen (besonders in späteren Stadien der Krankheit), wobei der Patient schlagartig in Bewegungsstarre verfällt.

Die Forderung nach einer verbesserten Darreichungsform mit verlängerter Wirkung, bei der zum Beispiel die on-off-Symptomatik verringert ist, wird daher seit langer Zeit erhoben (siehe S.M. Stahl, New Drug delivery systems - a new approach to Parkinson's disease, Symposium Harlow/GB 8.7.1985).

Aufgabe der Erfindung ist also die Bereitstellung eines verbesserten Arzneimittels aus Decarboxylasehemmer und L-Dopa mit verlängerter und verbesserter Wirkung für die Behandlung der Parkinsonschen Krankheit.

Es wurde nun überraschend gefunden, daß bei Verwendung einer Kombination aus Decarboxylasehemmer und L-Dopa-Pellets, wobei die Pellets auch eine verzögerte Freisetzung (eine sogenannte Retardierung) des Wirkstoffs aufweisen können, eine verlängerte und verbesserte Wirkung bei der Behandlung der Parkinsonschen Krankheit, insbesondere in deren fortgeschrittenen Stadien, zu beobachten ist.

So wird gegenüber der bisher üblichen Behandlung mit der Kombination aus Decarboxylasehemmer und L-Dopa beispielsweise folgende Verbesserung erzielt: Die Patienten müssen weniger häufig die retardierten Kombinationen als die Standard-Kombinationen einnehmen, bei gleicher bzw. verbesserter Antiparkinsonwirksamkeit. Durch die verzögerte Resorption werden im Serum Spitzenkonzentrationen und die damit verbundenen Nebenwirkungen (Dyskinesien) vermieden. Mit der Retardkombination werden wirksame L-Dopa-Serumkonzentrationen über längere Zeit aufgebaut als mit der Standardkombination. Dadurch und durch die geringere Applikationsfrequenz mit der Retardkombination läßt sich die therapeutische L-Dopa-Dosis kumulativ reduzieren. Da das L-Dopa-Nebenwirkungssyndrom kumulativ-dosisabhängig ist, läßt sich mit der Retardkombination diese Langzeit-Nebenwirkung ebenfalls reduzieren.

Die Verbesserung und Verlängerung der Wirkung der erfindungsgemäßen Kombination läßt sich gegenüber der Standardkombination folgendermaßen nachweisen: Nach abendlicher Gabe der Kombination an Parkinson-Patienten werden die nächtlichen Bewegungen der Patienten durch Accelerometrie von Armen und Beinen gemessen. Parkinson-Patienten beginnen im Stadium der Akinesie ihre Versuche zur Bewegung mit den Beinen. Ein Wechsel der Bewegung von den Beinen zu den Armen zeigt eine Verbesserung des klinischen Bildes des Patienten an.

Ein weiterer Parameter für die verbesserte Wirksamkeit der erfindungsgemäßen Kombination gegenüber der Standardkombination ist nach abendlicher Gabe der Kombination die verringerte morgendliche Akinesie.

Aus der US-Patentschrift 3 557 292 ist unter anderem auch eine Kombination von L-Dopa und Benserazid bekannt, wobei jedoch das L-Dopa ebenfalls nicht in Pelletform vorliegt. Gegenüber dieser bekannten Kombination besitzt die erfindungsgemäße Kombination überraschenderweise folgende Vorteile: Geringere Applikationshäufigkeit, Verringerung der Nebenwirkungsrate, gleichmäßigere Wirkung.

Weiterhin ist aus der Deutschen Offenlegungsschrift 3 232 873 eine Kombination bekannt von L-Dopa mit den Decarboxylasehemmern Carbidopa und Benserazid. Auch hier wird L-Dopa nicht in Pelletform verwendet. Der Nachteil dieser bekannten Kombination gegenüber der erfindungsgemäßen Kombination ist, daß mit der bekannten Kombination eine nur unzureichende Antiparkinson-Wirksamkeit festzustellen ist, insbesondere die Dyskinesien der Patienten verlängert sind.

Die Erfindung betrifft ein Kombinationspräparat ausgenommen Zweischichtpellets, welches als Wirkstoff L-Dopa in Form von Pellets und Decarboxylasehemmer oder Salze dieser Verbindung mit physiologisch unbedenklichen Säuren bzw. Basen enthält, wobei auf 1 Gewichtsteil Decarboxylasehemmer 0,5 - 100 Gewichtsteile L-Dopa kommen, sowie die Verwendung dieses Kombinationspräparates zur Behandlung der Parkinsonschen Erkrankung.

Die in den Patentansprüchen angegebenen Gewichtsmengen beziehungsweise Gewichtsteile beziehen sich jeweils auf die reinen Wirkstoffe, das heißt nicht auf Salze dieser Wirkstoffe. Falls Salze verwendet werden, ändern sich die Mengen entsprechend dem geänderten Molgewicht der Salze.

Das Benserazid wird vorzugsweise als Säureadditionssalz verwendet, wobei insbesondere die Salze mit Halogenwasserstoffsäuren (zum Beispiel das Hydrochlorid, Hydrobromid) oder auch mit organischen Säuren (zum Beispiel Embonsäure, Maleinsäure, Citronensäure, Weinsäure) in Frage kommen. Carbidopa und L-Dopa werden im allgemeinen nicht in Form des Salzes verwendet. Falls L-Dopa als Salz eingesetzt wird, handelt es sich beispielsweise um ein Salz mit physiologisch verträglichen Alkali- oder Erdalkalimetallen.

Die Tagedosen der erfindungsgemäßen Kombination bestehen zum Beispiel aus 10 bis 800 mg, vorzugsweise 20 bis 300 mg und insbesondere 75 bis 250 mg Decarboxylasehemmer und 50 bis 8 000 mg, vorzugsweise 100 bis 3 000 mg, insbesondere 300 bis 1 500 mg L-Dopa.

Die Tagedosen können in Form einer einmaligen Verabreichung der gesamten Menge oder in Form von 1 bis 10 insbesondere 2 bis 8 Teildosen pro Tag gegeben werden. Im allgemeinen ist eine Verabreichung 3 bis 6mal, insbesondere 4 bis 5 mal täglich bevorzugt. Beispielsweise beträgt die bevorzugte Dosis für die Kombination von Decarboxylasehemmer und L-Dopa 25 bis 50 mg Decarboxylasehemmer und etwa 100 bis 500 mg L-Dopa 2 bis 6 mal täglich. Insbesondere beträgt diese Dosis etwa 25 mg Decarboxylasehemmer und etwa 100 mg L-Dopa 3 bis 5 mal täglich.

Decarboxylasehemmer und L-Dopa werden erfindungsgemäß beispielsweise in folgenden Gewichtsverhältnissen verwendet:
1 Gewichtsteil Decarboxylasehemmer wird zum Beispiel mit 0,5 bis 100 Gewichtsteilen L-Dopa, vorzugsweise 1 Gewichtsteil Decarboxylasehemmer mit 1 bis 50 Gewichtsteilen L-Dopa, insbesondere 1 Gewichtsteil Decarboxylasehemmer 2 mit 20 Gewichtsteilen L-Dopa verwendet beziehungsweise kombiniert.

Beispielsweise lassen sich für die Kombination 50 bis 1 000 mg L-Dopa und 10 bis 100 mg Decarboxylasehemmer, vorzugsweise 100 bis 500 mg L-Dopa und 25 bis 50 mg Decarboxylasehemmer leicht zum Arzneimittel formulieren.

Die Dosierungseinheit der erfindungsgemäßen Kombination kann beispielsweise enthalten: 10 bis 100 mg Decarboxylasehemmer, vorzugsweise 10 bis 50 mg, insbesondere 25 bis 50 mg Decarboxylasehemmer und 50 bis 1 000 mg, vorzugsweise 50 bis 500 mg, insbesondere 100 bis 500 mg L-Dopa. Diese Dosen können beispielsweise 1 bis 8, vorzugsweise 2 bis 6, insbesondere 3 bis 5 mal täglich verabreicht werden.

Selbstverständlich können auch galenische Zubereitungen hergestellt werden, welche die angegebenen Dosierungseinheiten 2 bis beispielsweise 5 mal enthalten.

Die in den vorangegangenen Seiten angegebenen Dosen und Gewichtsteile, die sich auf die Anwendung am Menschen beziehen, sind jeweils bezogen auf die freien Basen, beziehungsweise freien Säuren.

Unter Pellets sind kugel- oder zylinderartige Formlinge zu verstehen mit einem Durchmesser zwischen 0,1 und 2 mm. Sie werden hergestellt, indem geeignete Pulvermischungen mit Tablettenpressen, Kompaktoren oder Gummilochplatten verpreßt oder durch Zugabe von Lösungen oder Lösungsmitteln angeteigt, die entstehende plastische Masse durch Lochscheiben gedrückt, die entstehenden Stränge zerteilt, ausgerundet und getrocknet werden.

Eine andere Möglichkeit der Herstellung besteht im gleichzeitigen oder nacheinander erfolgenden Aufbringen der Wirkstoffe mit oder ohne Bindemittel auf Neutralpellets ohne Wirkstoff (sogenannte Nonpareilles).

Eine weitere Möglichkeit besteht in der Bindung des Wirkstoffs L-Dopa an Ionenaustauscher, zum Beispiel durch Bindung von L-Dopa an physiologisch verträgliche Ionenaustauscher. Als solche Ionenaustauscher können zum Beispiel eingesetzt werden: Acryl- und Methacrylharze mit austauschbarem Proton, das heißt sauren, insbesondere schwach sauren Gruppen wie COO^{⊖} (zum Beispiel Amberlite® IRP-64); Polystyrolharze mit austauschbarem Na⁺, saure Gruppen: SO₃^{⊖} (zum Beispiel Amberlite® IRP-69).

Bei den Ionenaustauschern handelt es sich um saure Ionenaustauscher. Das Maximalverhältnis L-Dopa : Ionenaustauscher beträgt etwa 1:1, das Minimalverhältnis etwa 1 Gewichtsteil L-Dopa auf 800 Teile Ionenaustauscherharz. Vorzugsweise werden auf 1 Gew.-Teil L-Dopa 1 bis 400 Gew.-Teile Ionenaustauscher, ganz besonders bevorzugt 1 bis 100 Gew.-Teile Ionenaustauscher verwendet. Die Bindung des L-Dopa erfolgt, indem man eine L-Dopa-Lösung durch ein Bett des Ionenaustauschers in einer Säule laufen lässt. Der beladene Ionenaustauscher wird getrocknet bei Temperaturen bis zu etwa 50°C. Vorzugsweise werden die beladenen Ionenaustauscherpartikel noch mit einer Umhüllung versehen, wie es beispielsweise in US-A-4,221,776 beschrieben ist. Ein Vorteil des zusätzlichen Einhüllens besteht darin, daß sich die Freisetzungsrate des Wirkstoffes durch die Wahl des Hüllenmaterials verändern und beeinflussen lässt. Das Trocknen der mit Hülle versehenen beladenen Ionenaustauscherpartikel kann mit Warmluft von 70°C bis 90°C erfolgen.
Die beladenen Ionenaustauscher können dann beispielsweise in Hartgelatinekapseln gefüllt werden.

Auch durch Vertropfen von Schmelzen fettartiger Substanzen, z.B. von Cetylstearylalkohol oder Wachsen können Pellets erhalten werden. Die hierzu eingesetzten Verfahren der Sprüherstarrung oder Vibrationsvertropfung sind in der Technik bekannt.

Die Herstellung der erfindungsgemäß eingesetzten Pellets erfolgt in der hierfür üblichen Weise.

Bevorzugt werden Pellets mit kontrollierter Freisetzung des Wirkstoffs verwendet, wobei es sich hierbei um Pellets handeln kann, die entweder nur L-Dopa enthalten oder Pellets, die sowohl L-Dopa als auch Decarboxylasehemmer enthalten. Die Pellets mit kontrollierter Freisetzung werden vorzugsweise dadurch erhalten, daß man in üblicher Weise hergestellte Pellets mit den genannten Wirkstoffen in bekannter Weise mit mindestens einem Hüllstoff überzieht. Als Hüllstoffe kommen in Frage: Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (z.B. Eudragit® RS), Copolymerisate aus Acryl- und Methacrylsäureestern und Trimethylammoniummethacrylat (z.B. Eudragit® RL); Polyvinylacetat; Fette, Öle, Wachse, Fettalkohole; Hydroxypropylmethylcellulosephthalat oder -acetatsuccinat; Cellulose-, Stärke- sowie Polyvinylacetatphthalat; Carboxymethylcellulose; Methylcellulose-phthalat, - succinat, -phthalatsuccinat sowie -phthalatsäurehalbester; Zein; Ethylcellulose sowie -succinat; Schellack; Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethyl-hexyl-acrylatmaleinsäureanhydrid; Crotonsäure-Vinylacetat-Copolymer; Glutaminsäure/Glutaminsäureester-Copolymer; Carboxymethylethylcelluloseglycerinmonooctanoat; Celluloseacetatsuccinat; Polyarginin.

Günstig ist es beispielsweise auch, 2 separate Hüllschichten zu verwenden; eine für die kontrollierte Freisetzung (wie zum Beispiel die oben genannten, wobei diese dann keine beziehungsweise nur wenig freie Carboxygruppen enthalten) und eine für die Magensaftresistenz, das heißt eine Hüllschicht, die die Freigabe im Magen verhindert. Insbesondere kommt dies für die reinen L-Dopa-Pellets in Frage. Als gesonderte Hüllstoffe für die Magensaftresistenz kommen die hierfür üblichen in Betracht, beispielsweise physiologisch verträgliche Polymere mit freien Carboxygruppen wie Copolymerisate der Acrylsäure und/oder der Methacrylsäure, Hydroxypropylmethylcellulosephthalat, Celluloseacetatphthalat, Methylcellulosephthalat und andere Phthalate.

Als Plastifizierungsmittel für diese Hüllstoffe kommen in Frage: Citronen- und Weinsäureester (Acetyltriethyl-, Acetyltributyl-, Tributyl- Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-phthalat), D-(2-Methoxy- oder ethoxyethyl)phthalat, Ethylphthalyl-, Butylphthalylethyl- und Butylglycolat; Alkohole (Propylenglykol, Polyethylenglykol verschiedener Kettenlängen), Adipate (Diethyl-adipat, Di(2-Methoxy- oder ethoxyethyladipat)); Benzophenon; Diethyl- und Dibutylsebacat, -succinat, -tartrat; Diethylenglykoldipropionat; Ethylenglykoldiacetat; -dibutyrat, -dipropionat; Tributylphosphat, Tributyrin; Hydroxypropylcellulose, Hydroxypropylmethylcellulose, Polyethylenglykolsorbitanmonooleat (Polysorbate wie Polysorbat 80); Sorbitanmonooleat, Polyvinylpyrrolidon.

Für die Hüllschicht können ein oder mehrere der genannten Hüllstoffe sowie ein oder mehrere der genannten Plastifizierungsmittel verwendet werden. Die Hüllschicht kann zusätzliche Substanzen zur Steuerung der Freisetzung des L-Dopa enthalten . Dies sind wasserlösliche Substanzen wie Polyethylenglykole, Polyvinylpyrrolidon, Copolymerisate aus Polyvinylpyrrolidon und Polyvinylacetat, Polyvinylacetat und ähnliche. Für denselben Zweck können aber auch die bereits erwähnten Plastifizierungsmittel Hydroxypropylcellulose und/oder Hydroxypropylmethylcellulose verwendet werden. In diesem Falle beträgt deren Menge beispielsweise 0,1 bis 5 Gewichts%, vorzugsweise 1 bis 3 Gewichts% bezogen auf die Hüllsubstanz. Das Aufbringen der Hüllschicht erfolgt durch Aufsprühen von Lösungen der genannten Substanzen in organischen Lösungsmitteln oder Suspensionen der genannten Substanzen in organischen Lösungsmitteln oder Wasser, wobei noch weitere Hilfsstoffe zur Optimierung der Verarbeitbarkeit zugesetzt sein können wie zum Beispiel oberflächenaktive Substanzen, Feststoffe wie Talkum und/oder Magnesiumstearat und/oder Pigmente.

Das Aufsprühen erfolgt zum Beispiel im Dragierkessel oder in perforierten Kesseln mit kontrollierter Führung des Trocknungsmediums oder im Luftsuspensionsverfahren; im allgemeinen wird bei Temperaturen zwischen 10°C und 80°C gearbeitet.

Bei der Herstellung von L-Dopa-Pellets durch Vertropfen einer Schmelze von L-Dopa in fettartigen Substanzen bzw. Wachsen kommen z.B. als solche Stoffen in Frage: Glyceride von gesättigten Fettsäuren C₈H₁₆O₂ bis C₁₈H₁₆O₂, hydriertes Erdnußöl, hydriertes Ricinusöl, hydriertes Baumwollsamenöl. Stearinsäure, Palmitinsäure, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome), Carnaubawachs, Bienenwachs, Fettalkohole (gerad- oder ververzweigtkettig) der Kettenlänge C₈H₁₇OH bis C₃₀H₆₁OH, insbesondere C₁₂H₂₅OH bis C₂₄H₄₉OH.

Bei der Herstellung durch Aufbringen auf Neutralpellets kommen als Bindemittel zum Beispiel in Frage: Gelatine, Gummi arabicum, Stärkekleister, Cellulose-Derivate (Methylcellulose, Carboxymethylcellulose, Hydroxyethylcellulose, Hydroxypropylcellulose), Natriumalginat, Pektin, Tragant, Polyvinylpyrrolidon, Polyvinylacetat, Polyvinylalkohol, Copolymerisat aus Vinylpyrrolidon und Vinylacetat.

Die Pellets können aber auch hergestellt werden durch Einbettung von L-Dopa in folgende Substanzen oder Mischungen folgender Substanzen:
- Verdauliche Fette, z.B.
   Triglyceride von gesättigten Fettsäuren C₈H₁₆O₂ bis C₁₈H₃₆O₂ und deren Gemische, Erdnußöl und hydriertes Erdnußöl, Ricinusöl und hydriertes Ricinusöl, Olivenöl, Sesamöl, Baumwollsamenöl und hydriertes Baumwollsamenöl, Maisöl, Weizenkeimöl, Sonnenblumensamenöl, Kabeljau-Leberöl, Gemische von Mono-, Di-, Triestern der Palmitin- und Stearinsäure mit Glycerin, Glycerintrioleat, Diglykolstearat, Stearinsäure,
- Unverdauliche Fette bzw. fettähnliche Substanzen, z.B. Ester von aliphatischen gesättigten oder ungesättigten Fettsäuren (2 bis 22 C-Atomen, insbesondere 10 bis 18 C-Atome) mit einwertigen aliphatischen Alkoholen (1 bis 20 C-Atome), Carnaubawachs, Bienenwachs, Fettalkohole (geradkettig oder verzweigtkettig) der Kettenlänge C₈H₁₇OH bis C₃₀H₆₁OH, insbesondere C₁₂H₂₅OH bis C₂₄H₄₉OH.
- Polymere wie
   Polyvinylalkohol, Polyvinylchlorid, Polyacrylsäure; anionische Polymerisate aus Methacrylsäure und Methacrylsäureestern (Eudragit®L, Eudragit®S), Acryl- und Methacrylsäureester-Copolymerisate mit Trimethylammoniummethacrylat (Eudragit® RL, Eurdragit® RS), Copolymerisat von Acrylsäureethyl- und Methacrylsäuremethylestern Eudragit® NE 30 D), sowie aus Acrylsäure, Methacrylsäure sowie deren Estern (Verhältnis der freien Carboxygruppen zu den Estergruppen 1:1) (Eudragit® L 30 D), Polyethylen, Polyglycolsäure, Polyhydroxybuttersäure, Polymilchsäure, Copolymere aus Milchsäure und Glycolsäure (Hersteller: Boehringer Ingelheim), Copolymere aus Milchsäure und Ethylenoxid sowie Glycolsäure und Ethylenoxid, Hydroxypropylmethylcellulose-phthalat oder -acetatsuccinat; Celluloseacetatphthalat, Stärkeacetatphthalat, sowie Polyvinylacetatphthalat; Carboxymethylcellulose; Methylcellulose-phthalat, -succinat, -phthalatsuccinat, Methylcellulose-phthalsäurehalbester; Zein; Ethylcellulose; Schellack, Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethylhexylacrylat-maleinsäureanhydrid; Crotonsäure-Vinylacetat-Copolymer; Glutaminsäure/Glutaminsäureester-Copolymer; Carboxymethylcellulose-glycerinmonooctanoat; Celluloseacetatsuccinat; Polyarginin.
- Quellstoffe wie Methylcellulose, Hydroxypropylcellulose, Hydroxypropylmethylcellulose (Pharmacoat, Methocel E = Propylenglykoläther der Methylcellulose), Alginsäure und ihre Salze (Na-, Ca-Salz, auch Mischungen aus Alginsäure und Calciumsalzen z.B. CaHPO₄), Stärke, Carboxymethylstärke, Carboxymethylcellulose und deren Salze, (z.B. Na-Salz), Galaktomannan, Gummi arabicum, Karaya Gummi, Ghatti Gum, Agar-agar, Carrageen, Xanthan-Gummi, Guar Gummi und seine Derivate, Johannisbrotkernmehl, Propylenglykolalginat, Pektin, Traganth.

Insbesondere kommt beispielsweise auch eine Einbettung von L-Dopa und gegebenenfalls dem Decarboxylasehemmer in hydrophile Polymere oder Hydrocolloide, gegebenenfalls mit weiteren Hilfsstoffen, in Frage.
Solche hydrophilen Polymere oder Hydrocolloide sind in Wasser lösliche oder quellbare Stoffe, wie Cellulosederivate und Gummen. Das Hydrocolloid enthält vorzugsweise Cellulosederivate, nämlich Celluloseether, wie Methylcellulose, Cellulosealkylhydroxylate, wie Hydroxypropylmethylcellulose, Hydroxypropylcellulose, Hydroxymethylcellulose oder Hydroxyethylcellulose; Cellulosealkylcarboxylate wie Carboxymethylcellulose oder Carboxyethylcellulose und Alkalimetallsalze von Cellulosealkylcarboxylate, wie Natriumcarboxymethylcellulose und Natriumcarboxyethylcellulose oder Acrylsäure-Homopolymerisate oder - Copolymerisate oder deren Alkalimetallsalze.

Das Molekulargewicht und der Grad der Ethersubstitution des Celluloseethers sind nicht kritisch, und alle Handelsprodukte können in dieser Erfindung verwendet werden. Der verwendete Celluloseether hat im allgemeinen eine Viskosität von 3 bis 100 000, vorzugsweise 3 bis 10 000, und besonders bevorzugt 6 bis 6000 Centipoises, bestimmt an einer wäßrigen 2-gewichtsprozentigen Lösung bei 20°C. Ferner hat der verwendete Celluloseether im allgemeinen einen Ethersubstitutionsgrad von 0,1 bis 6 und vorzugsweise von 0,4 bis 4,6.
Der Grad der Ethersubstitution gibt die Durchschnittsanzahl an Ethergruppen für 3 Hydroxylgruppen pro Glucoseeinheit der Cellulose an.
Das verwendete Acrylsäure-Copolymerisat kann ein Copolymerisat aus Acrylsäure mit Allylsaccharose, Methylacrylat, Methacrylsäure, Methylmethacrylat, Hydroxyethylmethacrylat, Styrol oder einem Monomer eines Vinylethers, wie Methylvinylether sein.
Der Anteil des Comonemers kann innerhalb des Bereichs variiert werden, indem das Copolymerisat in Wasser löslich oder quellbar ist. Der Anteil ist allgemein nicht größer als etwa 20 Mol%, bezogen auf das Copolymerisat.
Ein handelsübliches Gemisch aus Acrylsäure-Homopolymerisat oder -Copolymerisat mit einer geringen Menge (gewöhnlichen nicht mehr als 20 Gewichts%) eines anderen wasserlöslichen Polymers (zum Beispiel ein Methacrylsäurehomo- oder -mischpolymer oder dessen Salz oder Polyethylenglycol), kann ebenso verwendet werden.
Geeignete pharmakologisch verträgliche Salze der Acrylsäure-Homopolymerisate oder -Copolymerisate sind Alkalimetallsalze, wie Natrium- oder Kaliumsalze und Ammoniumsalze. Der Grad der Neutralisierung der Salze ist nicht beschränkt.

Die Acrylsäure-Hompolymerisate oder Copolymerisate oder ihre pharmakologisch verträglichen Salze können beliebige Molekulargewichte haben. Allgemein haben sie eine Viskosität von 360 bis 165 000, vorzugsweise von 3600 bis 16 500 Centipoises. Die Viskosität wird an einer 2,2-gewichtsprozentigen wäßrigen Lösung des Natriumsalzes mit einem pH-Wert von 7 bis 7,5 bei 25°C ± 0,5°C bestimmt.
Die Acrylsäure-Homopolymerisate oder -Copolymerisate oder ihre pharmakologisch verträglichen Salze können einzeln oder als Gemisch verwendet werden. In den Zubereitungen gemäß der Erfindung können auch andere bekannte Hydrocolloide verwendet werden, wie zum Beispiel Akaziengummi, Guargummi, Traganthgummi, Xanthangummi, Pectin, Ammonium oder Natriumalginat, Mischungen von Natrium- oder Ammoniumalginat mit physiologisch verträglichen Calcium-Salzen (zum Beispiel Calcium-Gluconat, - Hydrogenphosphat, -Chlorid) oder deren Gemische.
Bevorzugte Hydrocolloide sind Natriumcarboxymethylcellulose, Hydroxypropylmethylcellulose (wie Celacol HPM oder Methocel E oder K), Polyacrylsäure (wie Carbopol 934P), oder die zuvor erwähnten Alginate.

Die Herstellung dieser Darreichungsform kann erfolgen:
durch Lösen oder Dispergieren von L-Dopa oder seinen Salzen in den genannten Fetten oder fettähnlichen Substanzen oder Mischungen davon, auch unter Schmelzen der genannten Substanzen und anschließend Wiederabkühlen, Zerkleinern, evtl. Zufügen weiterer Substanzen wie z.B. die oben erwähnten wasserlöslichen oder in Wasser quellbaren Substanzen und Formung zu Pellets. Das Abkühlen der Schmelze und Zerkleinern kann auch in einem Schritt zusammengefasst werden, indem die Schmelze in kaltem Wasser dispergiert wird oder einer Spüherstarrung oder einer Vibrationsvertropfung unterworfen wird; durch Mischen von L-Dopa mit den genannten Fetten, Polymeren oder Quellstoffen oder Mischungen dieser Substanzen, auch unter Anwendung von Wärme, und Formung der Mischungen, evtl. nach Zusatz weitere Hilfsstoffe, zu Pellets;
durch Mischen von L-Dopa mit Lösungen der genannten Fette oder Polymeren in Wasser oder organischen Lösungsmitteln, wie z.B. Ethanol, Ethylacetat, Aceton oder Isopropanol, evtl. Mischen mit Trägermaterialien wie Cellulosen, sowie nachfolgendes Abdampfen der Lösungsmittel und Vermischen der erhaltenen Wirkstoffeinbettung mit weiteren Hilfsstoffen und Verarbeitung zu Pellets;
durch Anfeuchten einer Mischung aus L-Dopa und den genannten Quellstoffen mit organischen Lösungsmitteln wie Ethanol, Ethylacetat, Aceton oder Isopropanol, eventuell unter Beifügung von Bindemitteln, wie Polyvinylpyrrolidon oder Copolymeren aus Polyvinylpyrrolidon und Polyvinylacetat und anschließende Formung von Pellets, die anschließend getrocknet werden.

Die pharmazeutischen Zusammensetzungen beziehungsweise Arzneimittel enthalten als Wirkstoff die erfindungsgemäße Kombination in einer Formulierung. Die Einzelwirkstoffe der Kombination können aber auch in jeweils getrennten Formulierungen vorliegen, wobei die bereits angegebenen Wirkstoffmengen jeweils für die betreffende Dosierungseinheit verwendet werden. Die Wirkstoffe beziehungsweise die Wirkstoffkombination liegt gegebenenfalls in Mischung mit anderen pharmakologisch beziehungsweise pharmazeutisch wirksamen Stoffen vor. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können. Beispielsweise können die Pellets in Tabletten eingearbeitet werden, die im Magen beziehungsweise Darm zerfallen und hier die Pellets freisetzen.

Als Träger- und Hilfsstoffe für die pharmazeutischen Zubereitungen kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences Band 52 (1963), Seite 918 u. ff.,
H.v.Czetsch-Lindenwald, Hilfsstoffe für Pharmazie und angrenzende Gebiete; Pharm. Ind., Heft 2, 1961, Seite 72 u.ff.;
Dr. H.P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Polyvinylacetat, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat);Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl, Rizinusöl, Olivenöl, Sesamöl, Baumwollsaatöl, Maisöl, Weizenkeimöl, Sonnenblumensamenöl, Kabel-jau-Leberöl, jeweils auch hydriert; Mono-, Di- und Triglyceride von gesättigten Fettsäuren C₁₂H₂₄O₂ bis C₁₈H₃₆O₂ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen und Essigsäure, Benzylbenzoat, Dioxolane, Glyzerinformale, Tetrahydrofurfurylalkohol, Polyglykolether mit C₁-C₁₂-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose auch quervernetzt, oder mikrokristalline Cellulose.

Zur Herstellung von Lösungen oder Suspensionen kommen beispielsweise Wasser oder organische Lösungsmittel in Frage, wie zum Beispiel Methanol, Ethanol, Propanol, Isopropanol, Dichlormethan, Trichlorethan, Aceton, 1,2-Propylenglykol, Polyglykole und deren Derivate, Dimethylsulfoxid, Fettalkohole, Triglyceride, Partialester des Glycerins, Paraffine und ähnliche.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxid-Kondensationsprodukte von Fettalkoholen, Alkylphenolen oder Fettsäuren oder auch 1-Methyl-3-(2-hydroxyethyl)imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Ethylenoxid erhalten werden (zum Beispiel 40 Mol Ethylenoxid pro Mol Glycerid).

Beispiele für Oleotriglyceride sind Olivenöl, Erdnußöl, Rizinusöl, Sesamöl, Baumwollsaatöl, Maisöl. Siehe auch Dr. H.P. Fiedler, "Lexikon der Hilfsstoffe für Pharmazie, Kosmetik und angrenzende Gebiete" 1971, Seite 191 bis 195.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Die pharmazeutische und galenische Handhabung der Wirkstoffe erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80°C, vorzugsweise 20 bis 50°C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation kann in das Körperinnere erfolgen, beispielsweise oral, enteral.

Die erfindungsgemäße Kombination kann auch als ein Erzeugnis vorliegen, bei dem jeweils die beiden Einzelwirkstoffe in getrennten Formulierungen vorliegen, so daß auch eine getrennte oder auch zeitlich abgestufte Verabreichung möglich ist.

Falls solche getrennten Formulierungen vorliegen, sind diese aufeinander abgestimmt und enthalten die jeweiligen Wirkstoffe in der Dosierungseinheit in den Mengen und entsprechenden Gewichtsverhältnissen, in denen sie in der kombinierten Mischung vorliegen können.

Bei getrennter Anwendung ist es auch möglich, daß beide Kombinationspartner nicht gleichzeitig verabreicht werden. In solchen Fällen kann L-Dopa 5 bis 300 Minuten nach Verabreichung des Decarboxylasehemmers gegeben werden.

Die akute Toxizität der erfindungsgemäßen Kombination. ausgedrückt durch die LD 50, liegt bei oraler Applikation beispielsweise oberhalb 1700 mg/kg (gilt für verschiedene Tiere wie zum Beispiel Maus, Ratte).

### Beispiel 1: Kapseln mit 100 mg L-Dopa in Form von Pellets und 28,5 mg Benserazidhydrochlorid

2 000 g L-Dopa werden mit 220 g mikrokristalliner Cellulose gemischt und die Mischung mit einer Lösung von 60 g Polysorbat 80 in 820 g gereinigtem Wasser intensiv durchfeuchtet. Die feuchte Masse wird durch einen Extruder (Lochweite 0,8 mm) gegeben und die erhaltenen Stränge mit einem Spheronizer zerteilt und ausgerundet. Die erhaltenen feuchten Pellets werden in einem Wirbelschichttrockner bis auf eine relative Feuchtigkeit (Gleichgewichtsfeuchte) von 25-35 % getrocknet. Die getrockneten Pellets werden gesiebt. Nur die Siebfraktion von 0,5 bis 1,25 mm wird weiterverarbeitet.

1 600 g der Pellets werden mit einer Filmsuspension besprüht, die folgendermaßen hergestellt wird: 28 g Triethylcitrat werden mit 0,3 g Polysorbat 80 in 110 g gereinigtem Wasser emulgiert und die Emulsion mit 460 g einer 30 %igen Suspension von Copolymerisaten aus Acryl- und Methacrylsäureestern mit einem geringen Gehalt an Ammoniumgruppen (zum Beispiel Eudragit® RS 30 D) vermischt. 68 g Talkum werden in 515 g gereinigtem Wasser mit Hilfe einer üblichen Homogenisiereinrichtung suspendiert und die Suspension nach Zusatz von einigen Tropfen Silikon-Antischaumöl in die oben erhaltene Dispersion eingerührt.
Der Auftrag der so erhaltenen Suspension (Überzug für die retardierende Abgabe) auf die Pellets geschieht in üblicher Weise, zum Beispiel unter Verwendung eines Wirbelschichtsprühgranulators bei einer Zulufttemperatur von 40 - 50°C und einer Ablufttemperatur von maximal 40°C. Die Trocknung der Pellets erfolgt unter den gleichen Bedingungen. Es wird so viel von obengenannter Suspension aufgesprüht, bis das Gesamtgewicht der getrockneten Pellets 1 628 g beträgt.

Sodann werden 1 500 g Pellets der Siebfraktion unter 1,25 mm mit folgender Lacksuspension besprüht (Überzug für die Magensaftresistenz):
32 g Triethylcitrat werden mit 0,3 g Polysorbat 80 in 130 g gereinigtem Wasser emulgiert und die Emulsion mit 1 068 g einer Suspension eines Copolymerisats mit anionischem Charakter auf Basis von Poly(meth)acrylsäure und Poly(meth)acrylsäureestern (zum Beispiel Eudragit® L30D) vermischt. 160 g Talkum werden in 620 g gereinigtem Wasser mit Hilfe einer üblichen Homogenisiereinrichtung suspendiert und die Suspension nach Zusatz von einigen Tropfen Silikon-Antischaumöl in die oben erhaltene Dispersion eingerührt.
Der Auftrag der damit erhaltenen Suspension auf die Pellets geschieht wie oben angegeben. Es wird so viel von der Suspension aufgesprüht, bis das Gesamtgewicht der getrockneten Pellets 1 978 g beträgt.

570 g Benserazidhydrochlorid werden mit 420 g Lactose gemischt und mit einer Lösung von 20 g Gelatine in 180 g gereinigtem Wasser in üblicher Weise granuliert. Nach dem Trocknen und Sieben des Granulates durch ein Sieb der Maschenweite 0,8 mm werden 6 g Magnesiumstearat und 4 g Hochdisperses Siliciumdioxid zugemischt.

Die Mischung wird zu 51 mg zusammen mit jeweils 153 mg der oben erhaltenen lackierten Pellets in Hartgelatinekapseln der Größe 2 abgefüllt.

Eine Hartgelatinekapsel enthält 100 mg L-Dopa in Form von Pellets und 28,5 mg Benserazidhydrochlorid.

Die Freisetzung von L-Dopa aus dieser Darreichungsform wird geprüft nach dem Verfahren der US-Pharmakopöe, 21. Ausgabe (USP XXI) mit dem Gerät für den Dissolution-Test, Apparatur 2. Bei einer Umdrehungsgeschwindigkeit des Paddles von 120 Umdrehungen pro Minute wird die Freisetzung des L-Dopa in 900 ml Prüflösung bei 37°C bestimmt. Die Prüflösung besteht für die ersten 2 Stunden aus 0,06 molarer Salzsäure, danach werden die Pellets in Phosphatpufferlösung pH 6,8 der Europäischen Pharmakopoe überführt. Aus den Prüflösungen wird jeweils die Freisetzung des L-Dopa gemessen.

Die Freisetzung des L-Dopa beträgt

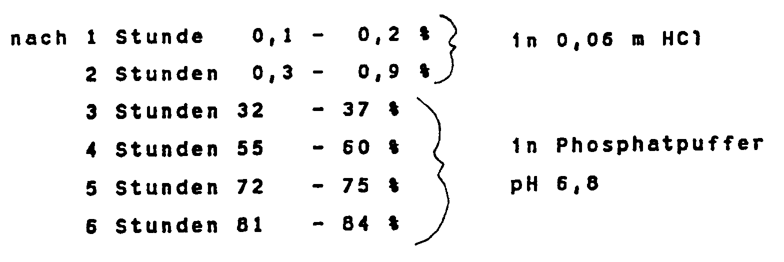

In ähnlicher Weise können Kapseln hergestellt werden, die neben der Granulatmischung aus Benserazidhydrochlorid
a) unlackierte L-Dopa-Pellets enthalten
b) L-Dopa-Pellets enthalten, die nur mit einer der genannten Suspensionen lackiert wurden
c) Mischungen aus unlackierten und lackierten Pellets enthalten.

Zum Erhalt dieser Pellets ist das Verfahren der Lackierung entsprechend abzuändern.

### Beispiel 2

### Kapseln mit 100 mg L-Dopa in Form von Mikrotabletten und 28,5 mg Benserazidhydrochlorid

In einem Mischer werden 9 kg L-Dopa mit 2,7 kg Methylhydroxypropylcellulose (Viskosität einer 2%igen Lösung: 15 000 cP), 9 kg Natriumalginat, 9 kg Calciumhydrogenphosphat und 0,06 kg Magnesiumstearat homogen gemischt. Die Mischung wird zu bikonvexen Tabletten von 2 mm Durchmesser und einer Dicke von 2 mm gepreßt. Die Tabletten werden in der üblichen Weise mit einem magensaftresistenten Film überzogen. Hierbei kann beispielsweise folgendermaßen verfahren werden:
In einer Lösung von 45 g Natriumcarboxymethylcellulose in 1000 g gereinigtem Wasser werden 24 g Titandioxid und 240 g Talkum homogen suspendiert.
Anschließend werden 54 g 1,2-Propylenglycol und 597 g Wasser hinzugefügt. Diese Suspension wird unter Rühren in 1500 g einer wäßrigen Dispersion eines anionischen Copolymerisates (50:50) auf Basis Methacrylsäure und Acrylsäuremethylester (Eudragit® L 30 D) eingetragen.

Auf 1 kg Tabletten rechnet man etwa 500 g der so hergestellten Lackiersuspension. Der Auftrag erfolgt zum Beispiel unter Verwendung eines Wirbelschichtgerätes bei einer Zulufttemperatur von 40-50°C und einer Ablufttemperatur von maximal 40°C.

570 g Benserazidhydrochlorid werden mit 420 g Lactose, 6 g Magnesiumstearat und 4 g hochdispersem Siliciumdioxid gemischt.

Diese Mischung wird zu je 50 mg zusammen mit jeweils 369 mg der zuvor erhaltenen lackierten Tabletten in Hartgelatinekapseln der Größe 0 abgefüllt.

Eine Hartgelatinekapsel enthält 100 mg L-Dopa in Form von pelletartigen Mikrotabletten und 28,5 mg Benserazidhydrochlorid.

Die Freisetzung von L-Dopa wird wie in Beispiel 1 geprüft nach dem Verfahren der USP XXI, jedoch bei einer Umdrehungsgeschwindigkeit des Paddles von 50 Umdrehungen pro Minute.

Die Freisetzung des L-Dopa beträgt

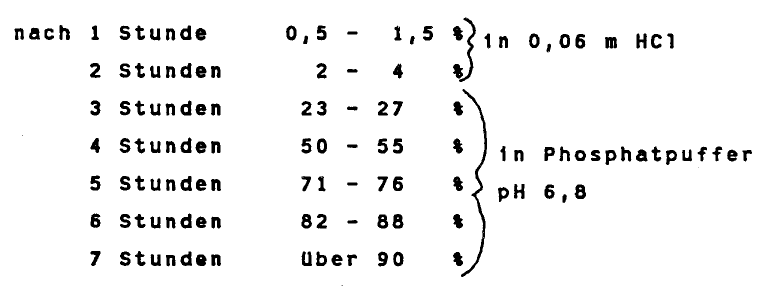

## Patentansprüche

1. Kombinationspräparat, ausgenommen in Form von Zweischichtenpellets, enthaltend als Wirkstoff L-Dopa in Form von Pellets und Decarboxylasehemmer oder Salze dieser Verbindungen mit physiologisch unbedenklichen Säuren bzw. Basen dadurch gekennzeichnet, daß für die Kombination auf 1 Gewichtsteil Decarboxylasehemmer 0,5 bis 100 Gewichtsteile L-Dopa kommen.

2. Kombinationspräparat nach Anspruch 1 dadurch gekennzeichnet, daß in der Dosierungseinheit für die Kombination 10-100 mg, vorzugsweise 25-50 mg, Decarboxylasehemmer und 50-1000 mg, vorzugsweise 100-500 mg, L-Dopa in Form von Pellets verwendet werden.

3. Kombinationspräparat nach Anspruch 1 dadurch gekennzeichnet, daß als Decarboxylasehemmer Benserazid oder Carbidopa eingesetzt werden.

4. Verfahren zur Herstellung eines peroral zu verabreichendes Kombinationspräparates nach Anspruch 1 dadurch gekennzeichnet, daß man 1 Gewichtsteil Decarboxylasehemmer und 0,5-100 Gewichtsteile L-Dopa, wobei die Wirkstoffe auch in Form von Salzen vorliegen können, zusammen mit üblichen Träger- und/oder Verdünnungs- beziehungsweise Hilfsstoffen bei Temperaturen zwischen 10 und 80°C vermischt oder homogenisiert, die so erhaltene Mischung zu Pellets mit einem Durchmesser zwischen 0,1 und 2 mm presst oder diese Mischung mit einem üblichen Lösungsmittel anteigt und die enstehende plastische Masse durch Lochscheiben drückt, die entstehenden Stränge zerteilt, ausrundet und trocknet oder die Mischung ohne Bindemittel auf Neutralpellets aufbringt, die Pellets gegebenenfalls mit einem Überzug versieht und diese in Kapseln oder Beutel entsprechender Größe abfüllt, so daß in der entsprechenden Dosierungseinheit 10-100 mg Decarboxylasehemmer und 50-1000 mg L-Dopa enthalten sind.

5. Verfahren zur Herstellung eines peroral zu verabreichendes Kombinationspräparates nach Anspruch 1 dadurch gekennzeichnet, daß man 0,5-100 Gewichtsteile L-Dopa, zusammen mit üblichen Träger- und/oder Verdünnungs- beziehungsweise Hilfsstoffen bei Temperaturen zwischen 10 und 80°C vermischt oder homogenisiert, die so erhaltene Mischung zu Pellets mit einem Durchmesser zwischen 0,1 und 2 mm presst oder diese Mischung mit einem üblichen Lösungsmittel anteigt und die enstehende plastische Masse durch Lochscheiben drückt, die entstehenden Stränge zerteilt, ausrundet und trocknet oder die Mischung ohne Bindemittel auf Neutralpellets aufbringt, die Pellets gegebenenfalls mit einem Überzug versieht und diese zusammen mit 1 Gewichtsteil Decarboxylasehemmer in Kapseln oder Beutel entsprechender Größe abfüllt, so daß in der entsprechenden Dosierungseinheit 10-100 mg Decarboxylasehemmer und 50-1000 mg L-Dopa enthalten sind.

6. Verwendung des Kombinationspräparates gemäß Anspruch 1 zur Herstellung eines Arzneimittels zur Behandlung der Parkinsonschen Erkrankung.

## Claims

1. Combination preparation, except in the form of double layer pellets, containing as active constituent L-dopa in the form of pellets and decarboxylase inhibitor or salts of these compounds with physiologically acceptable acids or bases, characterised in that 0.5 to 100 parts by weight of L-dopa are used per 1 part by weight of decarboxylase inhibitor for the combination.

2. Combination preparation according to claim 1, characterised in that 10-100 mg, preferably 25-50 mg of decarboxylase inhibitor and 50-1000 mg, preferably 100-500 mg of L-dopa in the form of pellets are used in the dosage unit for the combination.

3. Combination preparation according to claim 1, characterised in that Benserazid or Carbidopa are used as decarboxylase inhibitor.

4. Process for preparing a combination preparation according to claim 1 to be administered orally, characterised in that 1 part by weight of decarboxylase inhibitor and 0.5-100 parts by weight of L-dopa, the active constituents also being able to be present in the form of salts, are mixed or homogenised at temperatures between 10° and 80°C with conventional excipients and/or diluents and/or auxiliaries, the resulting mixture is compressed into pellets having a diameter of between 0.1 and 2 mm, or this mixture is made into a paste with a conventional solvent and the resultant plastic composition is forced through perforated plates, the resulting strands are separated, rounded off and dried, or the mixture is applied without a binder to neutral pellets and the pellets are optionally provided with a coating and packed in capsules or sachets of suitable size so that the corresponding dosage unit contains 10-100 mg of decarboxylase inhibitor and 50-1000 mg of L-dopa.

5. Process for preparing a combination preparation according to claim 1 to be administered orally, characterized in that 0.5-100 parts by weight of L-dopa are mixed or homogenized at temperatures between 10° and 80°C with conventional excipients and/or diluents and/or auxiliaries, the resulting mixture is compressed into pellets having a diameter of between 0.1 and 2 mm or this mixture is made into a paste with a conventional solvent and the resultant plastic composition is forced through perforated plates, the resultant strands are separated, rounded off and dried, or the mixture is applied without a binder to neutral pellets, the pellets are optionally provided with a coating, and these pellets together with 1 part by weight of decarboxylase inhibitor are packed into capsules or sachets of appropriate size so that the corresponding dosage unit contains 10-100 mg of decarboxylase inhibitor and 50-1000 mg of L-dopa.

6. Use of the combination preparation according to claim 1 to prepare a medication for treating Parkinson's disease.

## Revendications

1. Préparation combinée, sauf sous forme de pilules à deux couches, contenant en tant que substance active, de la L-dopa sous forme de pilules et un inhibiteur de décarboxylase ou des sels de ces composés avec des acides ou des bases sans inconvénient physiologique, caractérisée en ce que la L-dopa entre dans la combinaison à raison de 0,5 à 100 parties en poids pour 1 partie d'inhibiteur de décarboxylase.

2. Préparation combinée selon la revendication 1, caractérisée en ce qu'on on utilise, dans l'unité posologique pour la combinaison, de 10 à 100 mg, de préférence de 25 à 50 mg d'inhibiteur de décarboxylase et de 50 à 1000 mg, de préférence de 100 à 500 mg de L-dopa sous forme de pilules.

3. Préparation combinée selon la revendication 1, caractérisée en ce qu'on utilise comme inhibiteur de décarboxylase du bensérazide ou de la carbidopa.

4. Procédé de préparation d'une préparation combinée à administrer per os selon la revendication 1, caractérisé en ce qu'on mélange ou homogénéise, à des températures comprises entre 10 et 80°C, 1 partie en poids d'inhibiteur de décarboxylase et 0,5 à 100 parties en poids de L-dopa, ces substances actives pouvant aussi se présenter sous forme de sels, avec des excipients et/ou des diluants où produits auxiliaires usuels, on comprime le mélange ainsi obtenu en pilules ayant un diamètre de 0,1 à 2 mm ou on transforme ce mélange en pâte avec un solvant usuel et on fait passer la masse plastique résultante à travers des disques perforés, on divise les boudins formés, on les arrondit et on les sèche ou bien on applique le mélange sans liant sur des pilules neutres, on recouvre éventuellement les pilules d'un enrobage, et on charge celles-ci dans des capsules ou des sachets de dimensions appropriées, de telle sorte que soient contenus dans l'unité posologique correspondante 10 à 100 mg d'inhibiteur de décarboxylase et 50 à 1000 mg de L-dopa.

5. Procédé de préparation d'une préparation combinée à administrer per os selon la revendication 1, caractérisé en ce qu'on mélange ou homogénéise, à des températures comprises entre 10 et 80°C, 0,5 à 100 parties en poids de L-dopa, avec des excipients et/ou des diluants ou produits auxiliaires usuels, on comprime le mélange ainsi obtenu en pilules ayant un diamètre de 0,1 à 2 mm ou on transforme ce mélange en pâte avec un solvant usuel et on fait passer la masse plastique résultante à travers des disques perforés, on divise les boudins formés, on les arrondit et on les sèche ou bien on applique le mélange sans liant sur des pilules neutres, on recouvre éventuellement les pilules d'un enrobage, et on charge celles-ci avec 1 partie en poids d'inhibiteur de décarboxylase dans des capsules ou des sachets de dimensions appropriées, de telle sorte que soient contenus dans l'unité posologique correspondante 10 à 100 mg d'inhibiteur de décarboxylase et 50 à 1000 mg de L-dopa.

6. Utilisation de la préparation combinée selon la revendication 1 pour la préparation d'un médicament pour le traitement de la maladie de Parkinson.
